(19) Europäisches Patentamt
European Patent Office
Office européen des brevets

(11) Veröffentlichungsnummer: **0 003 981**
**B1**

(12) **EUROPÄISCHE PATENTSCHRIFT**

(21) Anmeldenummer: **79100455.9**

(22) Anmeldetag: **16.02.79**

(51) Int. Cl.³: **C 07 C 79/36, C 07 C 76/02**

---

(54) Verfahren zur Herstellung von 2-Nitrobenzaldehyd

---

(30) Priorität: **02.03.78 DE 2808930**

(43) Veröffentlichungstag der Anmeldung:
**19.09.79 Patentblatt 79/19**

(45) Bekanntmachung des Hinweises auf die Patenterteilung:
**07.01.81 Patentblatt 81/01**

(84) Benannte Vertragsstaaten:
**BE CH DE FR GB IT NL**

(56) Entgegenhaltungen:
**DE - A - 2 731 259**

**Chemical Abstracts vol. 54, no. 6, 1960
Columbus, Ohio, USA
N. KORNBLUM et al. "A new and selective
method of oxidation. Conversion of
alkyl halides and alkyl tosylates to aldehydes"**

**Chemical Abstracts vol. 63, no. 10, 1965
Columbus, Ohio, USA
S. TAKEO et al, "Photochemical
oxidation reaction of benzylic alcohols
in dimethyl sulfoxide"**

**Chemical Abstracts vol. 66, no. 15, 1967
Columbus, Ohio, USA
A. KALIR "o-Nitrobenzaldehyde"
Seite 6122, Abstract Nr. 65 228 n**

(73) Patentinhaber: **BAYER AG
Zentralbereich Patente, Marken und Lizenzen
D - 5090 Leverkusen 1, Bayerwerk (DE)**

(72) Erfinder: **Ertel, Werner, Dr.
Theodor-Heuss-Strasse 50
D - 5600 Wuppertal-11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Verfahren zur Herstellung von 2-Nitrobenzaldehyd

Die vorliegende Erfindung betrifft ein neues, fortschrittliches Verfahren zur Herstellung von 2-Nitrobenzaldehyd, welches als Zwischenprodukt vielseitig verwendbar ist und insbesondere bei der Herstellung von pharmazeutisch wirksamen 4-(1-Nitrophenyl)-1,4-dihydropyridinderivaten verwendet werden kann (vgl. DT-PS 1 670 827).

2-Nitrobenzaldehyd war bisher nur schwer zugänglich, da die meisten klassicchen Verfahren der Aldehydsynthese bei dieser Verbindung versagen. In der Deutschen Auslegeschrift 2 415 062 werden die Nachteile der literaturbekannten Verfahren ausführlich kommentiert. Das in dieser Auslegeschrift beschriebene Herstellungsverfahren aus 2-Nitrotoluol über die Zwischenstufen 2-Nitrophenylbrenztraubensäure und 2-Nitrobenzaldichlorid liefert zwar den 2-Nitrobenzaldehyd in guten Ausbeuten, ist aber als technisches Verfahren immer noch aufwendig, bedingt durch die Art und die Anzahl der Reaktions- und Reinigungsschritte.

Als weiterer Herstellungsweg ist in der DT-OS 27 08 115 die Hydrolyse von 2-Nitrobenzylbromid zum entsprechenden Alkohol und anschließende Oxidation mit verdünnter Salpetersäure beschrieben. Nachteile dieses Verfahrens sind der sehr hohe Zeitaufwand, da allein für die Hydrolyse 12 Stunden und für die Oxidation 5 Stunden Reaktionszeit benötigt werden, und das Entstehen von nitrosen Gasen, welches bei einer Salpetersäure-Oxidation unvermeidbar ist und zu besonderen Vorsichtsmaßnahmen zwingt.

Weiterhin ist es bekannt, Benzylhalogenide und Benzyltosylate mit Dimethylsulfoxid (DMSO) zu Aldehyden zu oxidieren (vgl. Kornblum et al., J.Am.Chem.Soc. *79* (1971), 6562). In dieser Publikation wird als Reaktionsbeispiel auch die Umsetzung von p-Nitrobenzylbromid in DMSO zu p-Nitrobenzaldehyd mit einer Ausbeute von 48% beschrieben. Höhere Ausbeuten an Aldehyden können erhalten werden, wenn man von den entsprechenden Tosylaten ausgeht (vgl. Kornblum et al., J.Am.Chem.Soc. *81* (1959), 4113—4).

Die vorliegende Erfindung betrifft ein neues fortschrittliches Verfahren zur Herstellung von 2-Nitrobenzaldehyd aus 2-Nitrotoluol, welches durch radikalische Bromierung zu 2-Nitrobenzylbromid umgesetzt wird, wobei das hierbei erhaltene Bromierungsöl mindestens 50% 2-Nitrobenzylbromid enthält, dadurch gekennzeichnet, daß man dieses Bromierungsöl ohne vorherige Isolierung des 2-Nitrobenzylbromides direkt mit einer Mischung von Dimethylsulfoxid und Natriumbicarbonat im Mischungsverhältnis 3:1 bis 10:1 bei Temperaturen bis 100°C oxidiert und anschließend in an sich bekannter Weise den 2-Nitrobenzaldehyd über das Bisulfit-Addukt isoliert.

Hierdurch erhält man überraschenderweise den 2-Nitrobenzaldehyd in großer Reinheit und mit hoher Ausbeute. Aufgrund der Ergebnisse von Kornblum hätte erwartet werden müssen, daß die Umsetzung von 2-Nitrobenzylbromid geringere Ausbeuten ergibt als sie bei der Herstellung von 4-Nitrobenzylbromid erhalten wurden (48%), da mit einer sterischen Behinderung der Reaktion durch die benachbare Nitrogruppe zu rechnen war, und da außerdem bei einer Isolierung von 2-Nitrobenzylbromid aus dem rohen Bromierungsöl mit einem starken Verlust zu rechnen war. Das 2-Nitrobenzylbromid ist wegen seiner geringen Stabilität nicht destillativ, sondern nur durch "Ausfrieren" zu isolieren.

Der 2-Nitrobenzaldehyd ist ein universell einsetzbares Ausgangsprodukt für die Herstellung zahlreicher Verbindungen und, wie die oben angeführten Schutzrechtsanmeldungen und Publikationen zeigen, besteht ein dringendes Bedürfnis, diese Verbindung auf einfache und wirtschaftliche Weise herzustellen. Die seit mehr als 20 Jahren bekannte Umsetzung von P-Nitrobenzylbromid mit DMSO zu p-Nitrobenzaldehyd führte offensichtlich bis heute aufgrund seiner geringer Ausbeute zu einem Vorurteil, welches durch die vorliegende Erfindung überwunden wurde.

Es konnte nicht erwartet weden, daß man bei Einsatz des rohen Bromierungsöls, anstelle des reinen 2-Nitrobenzylbromids, einen so glatten Reaktionsverlauf mit Ausbeuten von über 60% an 2-Nitrobenzaldehyd erhalten würde. Überraschend und vorteilhaft ist weiterhin die sehr kurze Reaktionszeit. Das Gemisch aus Bromierungsöl, DMSO und Bicarbonat wird im Verlauf von ca. 30 Minuten auf 100°C aufgeheizt, danach ist die Umsetzung beendet, und es kann sogleich mit der Aufarbeitung begonnen werden. Vorteilhaft ist auch die Möglichkeit, das DMSO destillative abzutrennen und mehrfach wieder als Oxidationsmittel zu verwenden.

Als weitere Vorteile sind zu nennen:

a) die Gefahr der Handhabung von 2-Nitrobenzylbromid (starke exotherme Zersetzung bei 110°C) wird durch den Einsatz des rohen Bromierungsöls, welches noch große Anteile von 2-Nitrotoluol enthält, beseitigt,

b) durch den schnellen Reaktionsablauf ist der Zeitaufwand gering,

c) die übrigen Umsetzungsprodukte der Reaktion, Wasser, $CO_2$, Natriumbromid und Dimethylsulfid, sind problemlos zu beseitigen.

Nach dem erfindungsgemäßen Verfahren ist es somit möglich, 2-Nitrobenzaldehyd in technisch einfacher, wirtschaftlicher und zeitsparender Weise herzustellen.

Das so erhaltene 2-Nitrobenzaldehyd läßt sich dann mit β-Dicarbonylverbindungen und Aminen zu pharmakologisch wirksamen 1,4-Dihydropyridinen weiter umsetzen (vgl. DT-OS 1 670 827).

Die Herstellung des Bromierungsöls bzw. des 2-Nitrobenzylbromids erfolgt zweckmäßigerweise durch radikalische Seitenkettenbromierung von 2-Nitrotoluol. Diese Reaktion kann sowohl unter UV-Licht, Infrarotlicht oder mit N-Brom-Succinimid oder durch Peroxidkatalysatoren erfolgen (vgl. DT-OS 26 14 484. Man erhält immer ein Gemisch von 2-Nitrobenzylbromid und nichtumgesetztem 2-Nitrotoluol. Dieses sogenannte Bromierungsöl hat in der Regel einen Gehalt an 2-Nitrobenzylbromid zwischen 50 und 75 Gew.—%.

Die folgende Bromierungsreaktion zeigt beispielhaft die Herstellung des erfindungsgemäß einsetzbaren Bromierungsöls.

### Herstellungsbeispiel

Man läßt zu einer Mischung von 500 ml DMSO und 100 g Natriumbicarbonat unter Stickstoff 100 g "Bromierungsöl" zulaufen. Im Verlauf von 30 Minuten heizt man den Ansatz unter Rühren auf 100°C. Anschließend kühlt man auf 50°C ab und destilliert bei dieser Temperatur und 2 bis 5 mm Hg 350 bis 400 ml DMSO ab. Der Rückstand wird mit 500 ml Toluol verrührt. Man saugt voim Natriumbromid und nichtumgesetztem Natriumbicarbonat ab und wäscht den Filterkuchen mit 200 ml Toluol nach. Der Feststoff wird verworfen. Das Filtrate wird unter Rühren mit einer Lösung von 80 g Natriumpyrosulfit in 120 ml Wasser versetzt. Nach Zugabe von 120 g Eis wird die Mischung 1 Stunde intensive gerührt. Nach Zugabe weiterer 200 ml Wasser werden die Phasen getrennt. Die wäßrige Phase wird mit 200 ml Toluol versetzt und mit 45%iger Natronlauge auf pH 12 alkalisiert. Die Temperatur steigt dabei auf 35 bis 40°C an. Man trennt die Phasen, vereint die Toluolphasen, verwirft die wäßrige Phase, wäscht die Toluolphase mit 50 ml Wasser und trocknet sie danach über Magnesiumsulfat. Nach dem Absaugen von Trockenmittel dampft man die Lösung im Vakuum ein. Der Rückstand — ein gelbes Öl — erstarrt beim Abkühlen zum gelben festen 2-Nitrobenzaldehyd.

Ausbeute: 24 g;
Gehalt: 98%.

### Patentansprüche

1. Verfahren zur Herstellung von 2-Nitrobenzaldehyd aus 2-Nitrotoluol, welches durch radikalische Bromierung zu 2-Nitrobenzylbromid umgesetzt wird, wobei das hierbei erhaltene Bromierungsöl mindestens 50% 2-Nitrobenzylbromid enthält, dadurch gekennzeichnet, daß man dieses Bromierungsöl direkt mit einer Mischung von Dimethylsulfoxid und Natriumbicarbonat im Mischungsverhältnis 3:1 bis 10:1 bei Temperaturen bis 100°C oxidiert und anschließend in an sich bekannter Weise den 2-Nitrobenzaldehyd über das Bisulfit-Addukt isoliert.

2. Verfahren gemäß Anspruch 1, dadurch gekennzeichnet, daß man das Bromierungsöl mit einer Mischung von Dimethylsulfoxid und Natriumbicarbonat im Verhältnis 5:1 oxidiert.

### Claims

1. A process for the production of 2-nitrobenzaldehyde from 2-nitrotoluene, in which 2-nitrotoluene is converted, by bromination by a radical mechanism, to 2-nitrobenzyl bromide in the form of a bromination oil containing at least 50% of 2-nitrobenzyl bromide, characterised in that this bromination oil is oxidised directly with a mixture of dimethylsulphoxide and sodium bicarbonate in a weight ratio of 3:1 to 10:1 at temperatures of up to 100°C and the 2-nitrobenzaldehyde is subsequently isolated via the bisulphite adduct in a manner known per se.

2. A process according to claim 1, characterised in that the bromination oil is oxidised with a mixture of dimethylsulphoxide and sodium bicarbonate in a weight ratio of 5:1.

### Revendications

1. Procédé de fabrication de 2-nitrobenzaldéhyde à partir de 2-nitrotoluène qui, par bromuration radicalaire, réagit en bromure de 2-nitrobenzyle, l'huile de bromuration ainsi obtenue contenant au moins 50% de bromure de 2-nitrobenzyle, caractérisé en ce qu'on oxyde cette huile de bromuration directement avec un mélange de diméthylsulfoxyde et de bicarbonate de sodium dans le rapport de mélange de 3:1 à 10:1 à des températures allant jusqu'à 100°C, puis en ce qu'on isole de manière connue en elle-même la 2-nitrobenzaldéhyde en passant par le produit d'addition bisulfitique.

2. Procédé selon la revendication 1, caractérisé en ce qu'on oxyde l'huile de bromuration avec un mélange de diméthylsulfoxyde et de bicarbonate de sodium dans le rapport de 5:1.